# EUROPEAN PATENT APPLICATION

(11) **EP 3 981 412 A1**
(43) Date of publication of application: **13.04.2022**
(21) Application number: 20819014.0
(22) Date of filing: 12.05.2020
(51) Int. Cl.: A61K 31/70, A61K 36/00, A61K 36/8962, A23K 10/30, A23L 33/125

(54) **PREBIOTIC COMPOSITION FOR THE PREVENTATIVE TREATMENT OF INTESTINAL INFECTIONS IN LIVESTOCK**

(30) Priority: 04.06.2019 ES 201930498
(71) Applicant: García Torés, Alberto Alejandro, 47410 Olmedo Valladolid (ES)
(72) Inventor: García Torés, Alberto Alejandro, 47410 Olmedo Valladolid (ES)
(74) Representative: Ungria López, Javier
(86) International application number: PCT/ES2020/070304
(87) International publication number: WO 2020/245479

(57) **Abstract**

The present invention relates to three formulations for stimulating the intestinal microbiota of mammals, particularly livestock, and is characterised in that it comprises prebiotics extracted from beetroot, from milk and from Allium sativum, and extracts of medicinal plants. The three formulations of the invention are suitable for the preventative treatment of intestinal infections such as clostridiosis, intestinal parasitosis, cryptosporidiosis and coccidiosis, as well as infections caused by some types of E. coli bacteria, and metabolic diseases such as pre-eclampsia. In addition, the formulations of the invention are administered in different phases of an animal's development, from pregnancy to the end of its reproductive and productive life.

## Description

### SECTOR OF THE ART

The present invention, as expressed in the title of this specification, relates to three prebiotic formulations rich in their composition in fruit-oligosaccharides obtained from beetroot, galacto-oligosaccharides obtained from milk, *Allium sativum* and extracts of medicinal plants for the selective stimulation of the intestinal microbiota. Another object of the invention is the preparation and use thereof as stimulators of the intestinal microbiota, which are applied in different phases of the pregnancy and rearing of the offspring of animal livestock.

### BACKGROUND OF THE INVENTION

The main problem in the intestinal health of newborn animals is the appearance of a series of intestinal infections such as clostridiosis, intestinal parasitosis, cryptosporidiosis, coccidiosis, pre-eclampsia, as well as infections caused by some types of E. Coli bacteria that cause diarrhoea which in most cases causes the death of the animal due to dehydration. Furthermore, there are also other types of diseases that affect the development of livestock, such as pre-eclampsia, which generate many economic losses in the sector.

These diseases have traditionally been treated with the use of antibiotics, the use of which is restricted by health authorities.

For this reason, in the field of livestock, a trend in nutrition is starting that is aimed at replacing antibiotics in livestock feed with natural products that can be healthier and at the same time safer for the use of livestock for human food.

In this line, the regulations that have been applied both at the European and national level are aimed at the gradual elimination of the use of antibiotics in animal feed. In fact, as early as November 2011, the European Commission indicated that "*Antimicrobial resistance is a growing health problem, with about 25,000 patients dying per year in the EU from infections caused by drug-resistant bacteria, and related costs of over 1.5 billion euros in healthcare expenses and productivity losses. Today, on the eve of the European Antibiotic Awareness Day, the European Commission presents a comprehensive action plan on antimicrobial resistance, which unveils twelve specific actions to be implemented in close cooperation with the Member States".*

In the same line, John Dalli, European Commissioner for Health and Consumer Policy, stated: "*We need to take swift and decisive action if we do not want to lose antimicrobial medicines as essential treatment against bacterial infections in both humans and animals. The twelve specific actions that we present today can help limit the spread of bacterial resistance and help develop new antimicrobial treatments. Their success requires a joint effort from the EU, the Member States, healthcare professionals, industry, farmers and many others".*

Moreover, Máire Geoghegan Quinn, Commissioner for Research and Innovation, stated: *"Finding the next generation of antibiotics is crucial if we are to stay ahead of the curve in the face of bacteria and other pathogens which are resistant to drugs. Investment in research and innovation will mean the best possible care for patients, and the Commission is working with industry and EU Member States to make this a priority. This commitment will continue under Horizon 2020, our future funding programme for research and innovation".*

Following this European regulation, the action plan against bacterial resistance was established, including seven priority measures:
- Making sure antimicrobial agents are used appropriately both in human and veterinary medicine.
- Preventing microbial infections and their spread.
- Developing new effective antimicrobial agents or alternative treatments.
- Cooperating with international partners to contain the risks of bacterial resistance.
- Improving monitoring and surveillance in human and veterinary medicine.
- Research and innovation.
- Communication, education and training.

These measures were implemented through twelve specific actions, among which the following stand out:
- Strengthening EU law on veterinary drugs and on medicated feed.
- Introducing recommendations for prudent use of antimicrobial agents in veterinary medicine, including follow-up reports.
- Introducing new legal instruments in the new EU Animal Health Law to tighten prevention and control of infections in animals.
- Strengthening surveillance systems on bacterial resistance and the administration of antimicrobial agents in veterinary medicine.

On this basis, legislation is moving towards a more restricted use of antibiotics, avoiding the use of said medications for palliative purposes, which occurred frequently in the livestock sector, restricting them exclusively to their use in the treatment of a bacterial infection. In fact, in response to the Communique of the European Commission that asked the Member States for an Action Plan on Antimicrobial Resistance, the Interterritorial Council of the National Health System and the Intersectoral Conference on Agriculture of Spain approved the National Plan against Antibiotic Resistance (PRAN) in 2014, wherein six strategic lines were defined, highlighting the corresponding *"Identifying alternative and*/*or complementary measures for prevention and treatment".*

This fact has given rise to the need to seek out effective alternatives that guarantee the use of other compounds that prevent the spread of bacterial infections and improve not only the quality of life of livestock but also avoid an excessive use of this type of drugs, which can be a risk, ultimately, for human consumption.

The invention described herein proposes as a solution to the existing problem in the livestock sector the use of prebiotics to promote the development of the intestinal probiota which contributes to the development and strengthening of the immune system, thus eliminating the habitual use of antibiotics in the first life stages of livestock.

This fact is already known in the state of the art, as described in the following documents.

The scientific article "Effect of Probiotics/Prebiotics on Cattle Health and Productivity" Yutaka Uyeno, Suguru Shigemori and Takeshi Shimosato. Microbes Environ. Vol. 30. No. 2, 126-132, 2015, which describes the ability of probiotics and prebiotics to modulate the balance and activity of the microbiota residing in the intestine and, in particular, discusses their benefits in ruminants both at a nutritional level and in general health.

Patent application US2016120915 (A1), which describes the characterisation of changes in mammalian microbiota associated with antibiotic treatments and various immunological conditions and related therapeutic methods involving the use of probiotics, prebiotics, symbiotics and antibiotics. This particular document proposes the administration of what is called bacterial analogues, such as recombinant DNA from bacteria, or bacterial heterologous genes, extract from dead bacteria, etc.

Nevertheless, even with this knowledge, to date formulations similar to that of the present invention have not been used, nor have the surprising results described herein been observed.

Therefore, the object of the present invention, unlike the methods and formulations of prebiotics described in the state of the art, relates to three new formulations for selectively stimulating the intestinal microbiota through the symbiosis of prebiotics extracted from beetroot, from milk, from *Allium sativum* and medicinal plants. The aim of said formulations is to feed the livestock's own intestinal flora and without providing external probiotics.

Based on this, a response to the need that currently exists in the livestock sector is proposed, thus avoiding the preventative treatment of animals by means of antibiotics and providing a double solution. The first solution is to prevent bacterial diseases in livestock and prevent possible economic losses. The second solution is to avoid the possible indirect human consumption of preventative antibiotics that have had to be administered to livestock for their treatment using biosecure products that are easily digested by animals. Likewise, the preventative treatment object of the invention entails cost savings and a less invasive application by means of the use of natural remedies the use of which is beneficial not only for the ovine species, but which can also be extended to other livestock species such as goats, swine, etc.

### DESCRIPTION OF THE INVENTION

In this manner, a first object of the invention relates to three prebiotic formulations for selectively stimulating the intestinal microbiota of mammals, preferably livestock, characterised in that it comprises prebiotics extracted from beetroot, from milk, from *Allium sativum* and medicinal plant extracts.

What characterises the present invention with respect to other types of inventions is that three different formulations are proposed which are applied in different phases of an animal's development, from pregnancy to the end of its productive or reproductive life.

Therefore, the object of the present invention is the formulation 1 that is administered to the pregnant female during the pregnancy phase, which is also called the pregnancy formulation. Another object of the invention is the formulation 2 that is administered to the offspring during the colostrum feeding phase, which is also called birth formulation. And finally, another object of the present invention is the formulation 3 that is administered during the lactation phase, and which is also called lactation formulation.

The three formulations object of the invention share three common components that are prebiotics extracted from beetroot, from milk, from Allium sativum, better known by its common name, garlic.

Fructooligosaccharides, sometimes also called oligofructoses or oligofructans or abbreviated FOS, are a type of soluble fibre abundant in vegetables such as beetroot. A fructooligosaccharide is a linear oligosaccharide made up of between 10 and 20 fructose monomers, linked by β(1→2) bonds and which may contain an initial glucose molecule. In addition, the formulations object of the invention defined below comprise galactooligosaccharides, known in abbreviated form as GOS; they are ingredients of natural origin, used by the food industry as sweeteners, and bulking agents are derived from lactose in milk.

*Allium sativum* or garlic is a plant, the most common part of which is the bulb and, in particular, the garlic cloves. Its components include volatile oils (including allicin, allin and ajoin), enzymes, vitamin A, B, C and E, minerals (including selenium and germanium), flavonoids.

An object of the invention is therefore the pregnancy formulation or formulation 1 comprising the following components in proportion to the total weight of the composition:
- Beetroot molasses between 77.5-98% by weight;
- *Allium sativum* between 1-10% by weight;
- Fructo-oligosaccharides between 0.30-4.80% by weight;
- Galacto-oligosaccharides between 0.30-4.80% by weight;
- Natural additives between 0-10% by weight;
- Functional additives between 0.10-1.5% by weight;
   wherein the sum of all the components is less than or equal to 100%.

The pregnancy formulation or formulation 1 is suitable for administering to pregnant sheep 30 days before the birth of the lamb.

In a preferred embodiment of the present invention, the pregnancy formulation or formulation 1 comprising the following components in proportion to the total weight of the composition:
- Beetroot molasses between 80-94% by weight;
- *Allium sativum* between 3-8% by weight;
- Fructo-oligosaccharides between 1.20-3.80% by weight;
- Galacto-oligosaccharides between 1.20-3.80% by weight;
- Natural additives: 0-8% by weight;
- Functional additives between 0.40-1.20% by weight;
   wherein the sum of all the components is less than or equal to 100%.

In an even more preferred embodiment of the present invention, the pregnancy formulation or formulation 1 comprising the following components in proportion to the total weight of the composition:
- Beetroot molasses between 82-92% by weight;
- *Allium sativum* between 4-7.5% by weight;
- Fructo-oligosaccharides between 1.60-3.20% by weight;
- Galacto-oligosaccharides between 1.60-3.20% by weight;
- Natural additives 0-7%
- Functional additives between 0.70-1.10% by weight;
   wherein the sum of all the components is less than or equal to 100%.

The formulation 1 is rich in beetroot molasses, which is the concentrated liquid syrup that remains after repeated crystallisations during the refining process of the sugar from the sugar beetroot. Its appearance is a thick and opaque liquid, brown to dark brown in colour, with a peculiar odour of beetroot molasses, with a sweet taste and a bitter aftertaste. It has complete solubility in hot and cold water. Molasses is therefore used as a source of sugar, for which reason the consumption thereof is attractive to livestock, and it is easy to digest.

In a preferred embodiment, the formulation 1 can comprise a natural additive; it can be milk thistle up to a maximum of 8% by weight with respect to the total weight of the formulation 1, and in an even more preferred embodiment, the formulation 1 can comprise a natural additive; it can be milk thistle up to a maximum of 7% by weight with respect to the total weight of the formulation 1.

Another object of the present invention is the birth formulation or formulation 2 comprising the following components in percentage by weight with respect to the total weight of the composition:
- *Allium sativum* between 7-22% by weight;
- Fructo-oligosaccharides between 19-38% by weight;
- Galacto-oligosaccharides between 8-27% by weight;
- Propylene glycol between 0-15% by weight;
- Selenium between 9-30% by weight;
- Natural additives 0-0.10%
- Functional additives between 11.50-28.50% by weight;
   wherein the sum of all the components is less than or equal to 100%.

In a preferred embodiment of the present invention, the birth formulation or formulation 2 comprising the following components in percentage by weight with respect to the total weight of the composition:
- *Allium sativum* between 10-18% by weight;
- Fructo-oligosaccharides between 20-29% by weight;
- Galacto-oligosaccharides between 12-24% by weight;
- Propylene glycol between 2-10% by weight;
- Selenium between 10-27% by weight;
- Natural additives 0-0.09%
- Functional additives between 14-26% by weight;
   wherein the sum of all the components is less than or equal to 100%.

In an even more preferred embodiment of the present invention, the birth formulation or formulation 2 comprising the following components in percentage by weight with respect to the total weight of the composition:
- *Allium sativum* between 7.5-12.5% by weight;
- Fructo-oligosaccharides between 15-25% by weight;
- Galacto-oligosaccharides between 15-24% by weight;
- Propylene glycol between 3-8% by weight;
- Selenium between 15-25% by weight;
- Natural additives 0-0.08%
- Functional additives between 15-25% by weight;
   wherein the sum of all the components is less than or equal to 100%.

The birth formulation or formulation 2 is suitable for administering when the lambs have just been born and it is supplied together with their usual feed during the first 48 hours of life.

In a preferred embodiment, the formulation 2 can comprise as natural additives 0.01-0.07% by weight of propolis extract and 0.01-0.09% by weight of echinacea with respect to the total weight of the formulation 2, and in an even more preferred embodiment, the formulation 2 can comprise as natural additives 0.01-0.06% by weight of propolis extract and 0.01-0.08% by weight of echinacea with respect to the total weight of the formulation 2.

Another object of the invention is the lactation formulation or formulation 3 comprising the following components in percentage by weight with respect to the total weight of the composition:
- *Allium sativum* between 19-35% by weight;
- Fructo-oligosaccharides between 19-35% by weight;
- Galacto-oligosaccharides between 19-35% by weight;
- Apple cider vinegar between 8-40% by weight;
- Functional additives between 7-24% by weight;
   wherein the sum of all the components is less than or equal to 100%.

In a more preferred embodiment of the invention, the lactation formulation or formulation 3 comprising the following components in percentage by weight with respect to the total weight of the composition:
- *Allium sativum* between 21-32% by weight;
- Fructo-oligosaccharides between 21-32% by weight;
- Galacto-oligosaccharides between 21-32% by weight;
- Apple cider vinegar between 10-36% by weight;
- Functional additives between 10-21% by weight;
   wherein the sum of all the components is less than or equal to 100%.

In an even more preferred embodiment of the invention, the lactation formulation or formulation 3 comprising the following components in percentage by weight with respect to the total weight of the composition:
- *Allium sativum* between 23-29% by weight;
- Fructo-oligosaccharides between 23-29% by weight;
- Galacto-oligosaccharides between 23-29% by weight;
- Apple cider vinegar between 13-29% by weight;
- Functional additives between 14-19% by weight;
   wherein the sum of all the components is less than or equal to 100%.

The lactation formulation or formulation 3 is suitable for administering when the lambs are between 40 and 50 hours old and until the end of their productive and reproductive cycle. In the context of the present invention, an animal's productive and reproductive cycle ranges from the first birth to the discard or death of the animal.

All three formulations can comprise functional additives, which in the context of the present invention may be vitamins, provitamins, amino acids or the salts or analogues thereof and chemically defined substances having a similar effect. Said functional additives are selected from the group consisting of vitamin A (3a672a), Vitamin D3 (E-671), Vitamin E (3a700), Vitamin B1 (3a820), Vitamin B2 (riboflavin), Vitamin B6 (3a831), Vitamin B12 (cyanocobalamin), Vitamin C (3a300), Vitamin K3 (3a710), Folic Acid (3a316), Nicotinic acid (3a314), Lime pantothenate (3a841), D, L Methionine (3c301), L-Lysine (3.2.1), L-Tryptophan (3.4.1), L- Threonine (3.3.1), Glutamic Acid, Aspartic Acid, Serine, Proline, Glycine, Alanine, Cystine, Isoleucine, Leucine, Phenylalanine, Tyrosine, Histidine, Arginine, and a combination thereof.

In addition, the formulations described herein can further comprise other additives that are natural, which are to be referred to in the context of the present invention as natural additives. In a preferred embodiment, the additives that can comprise the three formulations object of the invention are selected from the group consisting of thyme, rosemary, milk thistle, marshmallow, mint, green anise, black tea, eucalyptus, flax, lemon balm, chamomile, lavender, angelica, yarrow, valerian, sage, blackthorn, boswellia, bramble, sagebrush, tarragon, mallow, oregano, aloe vera, dandelion, ginger, pumpkin seed, chia, sesame, cumin, caraway, fennel, coriander, boldo, cassia cinnamon, peppermint, wormwood, basil, horsetail, papaya, parsley, borage, guava, nopal, parietaria, marjoram, aloe, cilantro, liquorice, farfara, lobelia, helenium, mullein, lungwort, banana leaf, bay leaf, pollen, propolis, echinacea and a combination thereof. Likewise, in an even more preferred embodiment, said additives are selected from the group consisting of thyme, rosemary, milk thistle, mint, green anise, eucalyptus, lavender, oregano, aloe vera, fennel, peppermint, marjoram, bay leaf, pollen, propolis, echinacea and a combination thereof.

The three formulations object of the invention, as defined herein, in other words, the ingredients and the proportions thereof, give rise to a very surprising technical effect in the selective stimulation of the intestinal microbiota in livestock, which makes them suitable for this purpose. This is therefore considered to be a preventative treatment for a number of conditions that are common in livestock. In the context of the present invention, the conditions for which the three formulations object of the invention are effective are infections caused by the following organisms:
- E-Coli;
- Bacteria of the genus Clostridium;
- Intestinal parasites such as trematodes, cestodes, tapeworms, nematodes;
- Protozoa of the genus Cryptosporidium;
- Coccidia of the genus Eimeria or Isospora;

These infections cause severe diarrhoea that in most cases cause death due to dehydration mainly in newborn animals but also in animals in all their stages of development until the end of their life.

Likewise, the three formulations object of the invention, and in particular the formulation 1, are suitable for the preventative treatment of pre-eclampsia, which is a metabolic disorder characterised by hypoglycaemia, which is an abnormally low level of sugar in the blood, and hyperketonaemia, which is an abnormally increased level of ketone bodies in the blood, as a consequence of a negative energy balance. It appears during the last six weeks of pregnancy when nutritional needs are high and the ability to eat food is reduced mainly by the increase in the volume of the uterus.

It should be noted at this point that the three formulations described herein always have a highly surprising effect, selectively stimulating the intestinal microbiota of livestock, as can be seen in Figure 3 of this document, and when said formulations are administered consecutively at a certain development stage of the animals. For this reason, each of the formulations object of the invention is administered in the following terms:
- Formulation 1: It is administered to the pregnant mother 30 days before birth.
- Formulation 2: 0 hours to 48 hours of the animal's life.
- Formulation 3: From the first 48 hours of life until the end of the animal's productive and reproductive period.

It must be noted that the novel effect observed when administering the three formulations described herein together with the development stages of the animal in which they are administered is to avoid the preventative use of antibiotics, which is established as a common practice in the state of the art, and also to obtain really surprising results in the growth and development of the animals to which the formulations have been administered along with their usual diet.

The formulations object of the invention are optimal for use in animals of any type, since they can also be applied to domestic animals, such as dogs, cats or horses to name a few. Furthermore, however, in a preferred embodiment of the present invention, the three formulations are administered to livestock, and more preferably to ovine, bovine, swine or goat livestock, and can be used safely by employees in this sector, without the need for the intervention of a veterinarian or healthcare specialist, since the components of the present formulations object of the invention are entirely made up of natural components.

Another object of the invention is a dilution comprising any one of the formulations 1, 2 or 3, as described herein. Said dilution comprises the desired formulation diluted in water at a concentration comprised between 0.1% and 20% (v/v) and in a more preferred embodiment between 0.5% and 10% (v/v).

Another object of the invention is the method for manufacturing any of the formulations 1, 2 or 3 object of the invention which comprises mixing each of the components comprising each of the formulations as described herein and stirring gently, the stirring being between 100 and 200 rpm, for 5 to 8 minutes. Said obtention method is performed at room temperature. Taking into account that said method is performed in the facilities where livestock are cared for and said conditions may vary during the seasons of the year, without affecting in any way the preparation of any of the formulations or the storage thereof.

In the context of the present invention, room temperature can be comprised between 10 and 30 °C degrees taking into account the variation in temperature during the seasons of the year.

In addition, the components of the formulations described herein can be presented in both solid and liquid states, thus giving rise to a formulation in solid or liquid state, once the manufacturing process is finished. In this regard, it should be noted that the final presentation of the three formulations object of the invention does not affect in any way the technical effect that said formulations exert on livestock.

Another object of the invention is the use of the claimed formulations for the preventative treatment of intestinal infections such as clostridiosis, intestinal parasitosis, cryptosporidiosis, coccidiosis, as well as infections caused by some types of E. Coli bacteria, which cause diarrhoea that in most cases causes the death of newborn animals, and particularly, of livestock animals due to dehydration. In addition, the three formulations object of the invention, and in a particular manner, the formulation 1, are used for the preventative treatment of pre-eclampsia.

An object of the invention is therefore the three formulations for the preventative treatment of intestinal infections in livestock that are selected from the group consisting of clostridiosis, intestinal parasitosis, cryptosporidiosis, coccidiosis, infections caused by some types of E. Coli bacteria and combinations thereof. Another object of the invention is the formulation 1 for the preventative treatment of pre-eclampsia.

In addition, another object of the invention is the dilution described herein for the preventative treatment of intestinal infections in livestock that are selected from the group consisting of clostridiosis, intestinal parasitosis, cryptosporidiosis, coccidiosis, infections caused by some types of E. Coli bacteria and combinations thereof. Another object of the invention is the dilution of the formulation 1 for the preventative treatment of pre-eclampsia.

Therefore, in view of this document, it can be verified that the three formulations are used for the same purpose, in other words, the object of the present invention comprising the use of the three formulations at the different development stages of the animal gives rise to the technical effect that is the preventative treatment of the pathologies described in the previous paragraph, thus avoiding the prophylactic use of antibiotics.

Likewise, the main advantages derived from the technical features of the claimed composition are the following:
- first, the prophylactic use of antibiotics is avoided, which is in accordance with the European regulations of 2011 and the PRAN that was approved in Spain in 2014;
- likewise, animals that have undergone this prophylactic treatment have shown a higher growth rate than animals that have not received this treatment, as shown by the experimental results collected herein;
- moreover, it also entails a great economic advantage, since the loss of animals that can suffer intestinal infections such as clostridiosis, intestinal parasitosis, cryptosporidiosis, coccidiosis, as well as infections caused by some types of E. Coli bacteria is avoided,
- also, it entails a great economic advantage, since the loss of animals that can suffer pre-eclampsia is avoided;
- additionally, preventative treatment based on the administration of the claimed formulations has a much lower cost compared to the cost of antibiotics, which entails a substantial economic advantage;
- in addition, by being natural products, the staff administering the claimed formulations do not have to have any type of healthcare qualification, either as a healthcare or veterinary assistant for the administration thereof, also avoiding contact with materials that may be harmful to the operators who apply it, which can be a source of infection, and eliminating the animal health residues that the treatment with antibiotics generates; and
- finally, a much safer product for human consumption is obtained, by eliminating antibiotics given for prophylaxis from the diet of livestock.

### BRIEF DESCRIPTION OF THE FIGURES

To complement the description and make the invention more readily understandable, a set of drawings is attached as an integral part of said description, wherein the following has been depicted, with an illustrative and non-limiting character:
Figure 1 is an explanatory graph of the sample groups indicating the formulations object of the invention that the individuals have received during the study described in the preferred embodiment.
Figure 2 shows the average daily gain of lambs, expressed in grams, divided according to the intake of the formulations object of the invention during colostrum feeding and lactation.
Figure 3 is a graph showing the abundance of the 16 most abundant bacterial genera in lamb faeces.

### PREFERRED EMBODIMENT OF THE INVENTION

For the purpose of contributing to better understand the invention, and according to a practical embodiment thereof, an experiment is included as an integral part of this description wherein the three formulations are administered to a sample group of a flock of sheep.

### PREPARATION OF THE SAMPLE GROUP

The project was developed in a farrowing crate of the AGM Sheep Development Farm by means of the differentiated application to a group of animals divided according to the food and product supplied.

Starting from the created formula, the amounts of each of the components that have generated products have been adapted, being different for each feeding phase of the animals.

Thus, for the present preferred embodiment of the invention, the formulation 1 was administered to the pregnant female during the pregnancy phase. The formulation 2 was administered to the offspring during the colostrum feeding phase. And finally, the formulation 3 was administered during the lactation phase.

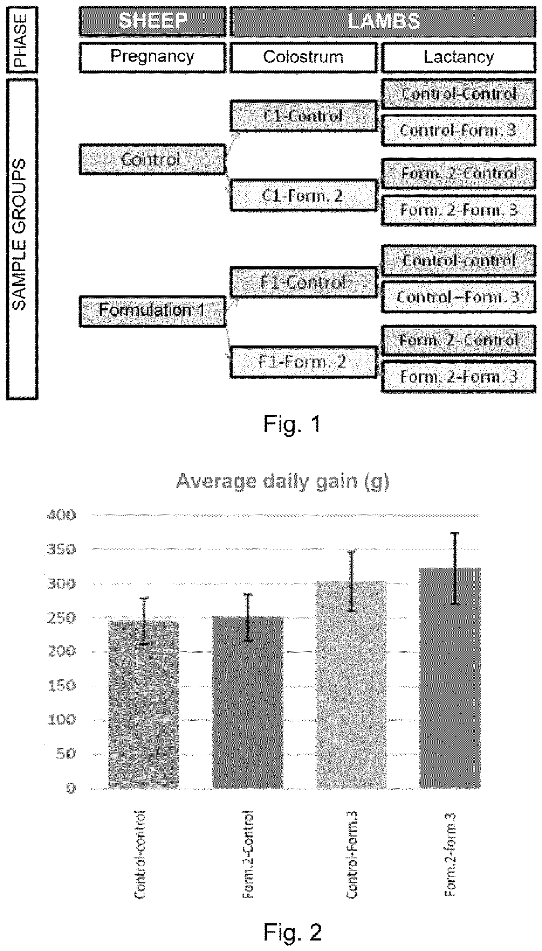

Figure 1 shows a diagram of the organisation of the groups of animals depending on the use or not of the formulations at each development stage of the animals.

The project started by forming two groups of mothers that were divided into:
- Control: Pregnant sheep to which the formulation 1 was not administered.
- Formulation 1: Pregnant sheep to which the formulation 1 was administered.

The lambs born from each group were in turn divided according to the colostrum received in their first 48 hours of life together with the formulation 2. Four divisions appear in the column "Colostrum" of Figure 1, which are explained below:
- C1-Control: Group with lambs born to mothers of the control group were fed colostrum without the formulation 2.
- C1-Form. 2: Group with lambs born to mothers of the control group fed with colostrum and given the formulation 2.
- F1-Control: Group of lambs born to mothers of the formulation 1 group were fed colostrum without the formulation 2.
- F1-Form. 2: Group of lambs born to mothers of the formulation 1 group fed with colostrum and given the formulation 2.

Lastly, the column "lactation" is composed of 8 groups that were fed with the formulation 3, after 48 hours, as follows:
- Control - Control: Lambs that have not received any formulation at any stage of their life and neither have their mothers.
- Control - Formulation 3: Lambs that have received the formulation 3 only in the last feeding phase.
- Formulation 2 - Control: Lambs whose mothers have not received the formulation 1, they have received the formulation 2 during the colostrum stage and they do not receive the formulation 3 during the lactation stage.
- Formulation 2 - Formulation 3: Lambs whose mothers are from the control group, they have received the formulation 2 during the colostrum stage and they also receive the formulation 3 during the lactation stage.
- Control - Control: Lambs whose mothers received the formulation 1, they did not receive the formulation 2 during the colostrum stage and they did not receive the formulation 3 during the lactation stage either.
- Control - Formulation 3: Lambs whose mothers received the formulation 1, they did not receive the formulation 2 during the colostrum stage and they did receive the formulation 3 during the lactation stage.
- Formulation 2 - Control: Lambs whose mothers received the formulation 1, they received the formulation 2 during the colostrum stage and they did not receive the formulation 3 during the lactation stage.
- Formulation 2 - Formulation 3: Lambs that have received all three formulations during all stages of their life as well as their mothers.

Figure 2 shows the increase in daily weight gain observed in those lambs that have taken the formulations for the longest time in their life. In addition, milk consumption was similar among the lambs that received the formulations object of the invention and those of the control group, so it has been concluded that the weight gain is not due to an increase in intake, but to the administration of the formulations object of the invention.

### ANALYSIS OF THE MICROBIOTA OF LAMBS

The first weeks of life are crucial for the establishment of the intestinal microbiota of ruminants and certain studies show that alterations in the intestinal microbiota due to diets during the first weeks of life have long-term effects.

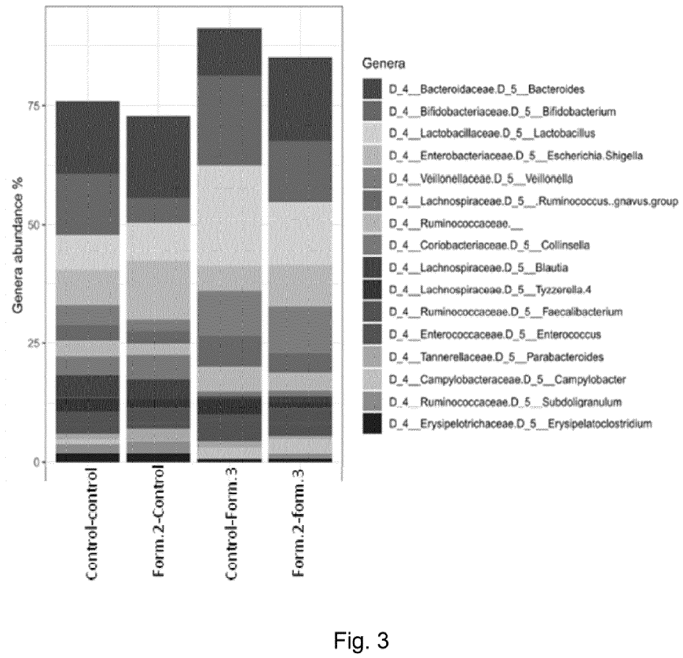

Figure 3 shows the 16 most abundant genera in the lamb samples. These genera account for more than 75% of the abundance and differences can be observed that have subsequently been confirmed as significant after the statistical study.

Bacteria of the genera Akkermansia, Bifidobacterium, Lactobacillus and Veillonella" were all significantly more abundant in those lambs that were given the formulation 3 with milk during the lactation or suckling phase. The presence of these 4 bacterial genera is considered crucial in the literature to be able to determine the good development of the lamb.

Especially Bifidobacterium, closely related to health benefits for lambs: regulation of homeostasis, inhibition of pathogens, modulation of immune responses both locally and systemically, production of vitamins and conversion of components of the diet into bioactive ingredients.

The lambs in the control-control group had higher abundances of Clostridiales, a common part of the intestinal microbiota, but methane generators, which is an effect that is not entirely desired since, although it helps to prevent carbon dioxide from accumulating in the gastrointestinal tract, methane is a greenhouse gas and is also related to a loss of energy/mass in ruminants.

For this reason, the present invention is presented as an effective alternative for the prevention of bacterial infections in livestock, and it is also a response to what is requested by the European regulations established since 2011.

## Claims

1. A formulation 1 for selectively stimulating the intestinal microbiota in livestock **characterised in that** it comprises, in percentage by weight:
• Beetroot molasses between 77.5-98% by weight;
• *Allium sativum* between 1-10% by weight;
• Fructo-oligosaccharides between 0.30-4.80% by weight;
• Galacto-oligosaccharides between 0.30-4.80% by weight;
• Natural additives between 0-10% by weight;
• Functional additives between 0.10-1.5% by weight;
wherein the sum of all the components is less than or equal to 100%.

2. A formulation 2 for selectively stimulating the intestinal microbiota in livestock **characterised in that** it comprises, in percentage by weight:
• *Allium sativum* between 7-22% by weight;
• Fructo-oligosaccharides between 19-38% by weight;
• Galacto-oligosaccharides between 8-27% by weight;
• Propylene glycol between 0-15% by weight;
• Selenium between 9-30% by weight;
• Natural additives 0-0.10%
• Functional additives between 11.50-28.50% by weight;
wherein the sum of all the components is less than or equal to 100%.

3. A formulation 3 for selectively stimulating the intestinal microbiota in livestock **characterised in that** it comprises, in percentage by weight:
• *Allium sativum* between 19-35% by weight;
• Fructo-oligosaccharides between 19-35% by weight;
• Galacto-oligosaccharides between 19-35% by weight;
• Apple cider vinegar between 8-40% by weight;
• Functional additives between 7-24% by weight;
wherein the sum of all the components is less than or equal to 100%.

4. A formulation 1 for selectively stimulating the intestinal microbiota in livestock according to claim 1, **characterised in that** it is administered to pregnant sheep during the last 30 days of the pregnancy period.

5. A formulation 2 for selectively stimulating the intestinal microbiota in livestock according to claim 2, **characterised in that** the formulation 2 is administered to newborn lambs and up to the first 48 hours of life.

6. A formulation 3 for selectively stimulating the intestinal microbiota in livestock according to claim 3, **characterised in that** the formulation 3 is administered to lambs that are between 40 and 50 hours old and until the end of their productive and reproductive life.

7. The formulation for selectively stimulating the intestinal microbiota in livestock according to claims 1 to 6, wherein the natural additive is selected from the group consisting of thyme, rosemary, milk thistle, marshmallow, mint, green anise, black tea, eucalyptus, flax, lemon balm, chamomile, lavender, angelica, yarrow, valerian, sage, blackthorn, boswellia, bramble, sagebrush, tarragon, mallow, oregano, aloe vera, dandelion, ginger, pumpkin seed, chia, sesame, cumin, caraway, fennel, coriander, boldo, cassia cinnamon, peppermint, wormwood, basil, horsetail, papaya, parsley, borage, guava, nopal, parietaria, marjoram, aloe, cilantro, liquorice, farfara, lobelia, helenium, mullein, lungwort, banana leaf, bay leaf, pollen, propolis, echinacea and a combination thereof.

8. The formulation for selectively stimulating the intestinal microbiota in livestock according to claims 1 to 7, wherein the functional additive is selected from the group consisting of vitamin A (3a672a), Vitamin D3 (E-671), Vitamin E (3a700), Vitamin B1 (3a820), Vitamin B2 (riboflavin), Vitamin B6 (3a831), Vitamin B12 (cyanocobalamin), Vitamin C (3a300), Vitamin K3 (3a710), Folic Acid (3a316), Nicotinic acid (3a314), Lime pantothenate (3a841), D, L Methionine (3c301), L-Lysine (3.2.1), L-Tryptophan (3.4.1), L- Threonine (3.3.1), Glutamic Acid, Aspartic Acid, Serine, Proline, Glycine, Alanine, Cystine, Isoleucine, Leucine, Phenylalanine, Tyrosine, Histidine, Arginine, and a combination thereof.

9. A formulation according to any one of claims 1 to 8, for the preventative treatment of intestinal infections in livestock that are selected from the group consisting of clostridiosis, intestinal parasitosis, cryptosporidiosis, coccidiosis, infections caused by some types of E. Coli bacteria and combinations thereof.

10. A formulation according to claims 1, 4, 7 or 8, for the preventative treatment of pre-eclampsia.

11. A dilution comprising a formulation according to claims 1 to 8, wherein said formulation is diluted in water at a concentration comprised between 0.1% and 20% (v/v).

12. A dilution according to claim 11, for the preventative treatment of intestinal infections in livestock that are selected from the group consisting of clostridiosis, intestinal parasitosis, cryptosporidiosis, coccidiosis, infections caused by some types of E. Coli bacteria and combinations thereof.

13. A dilution according to claim 11, for the preventative treatment of pre-eclampsia.

14. The method for manufacturing a formulation according to claims 1, 2 or 3, **characterised in that** it comprises:
a) mixing the components of a formulation according to claim 1 or claim 2 or claim 3,
b) stirring between 100 and 200 rpm, for 5 to 8 minutes and at a temperature comprised between 10 and 30 °C.

15. The method for manufacturing a formulation according to claims 10 to 12, wherein the components can be in solid or liquid state.
